# EUROPEAN PATENT APPLICATION

(11) **EP 2 510 811 A2**
(43) Date of publication of application: **17.10.2012**
(21) Application number: 12175365.1
(22) Date of filing: 01.11.2007
(51) Int. Cl.: A23L 1/29, A23L 1/30, A61K 31/201, A61K 38/02, A61K 45/06, A61K 31/202

(54) **Use of nutritional compositions for preventing disorders**

(30) Priority: 02.11.2006 WO PCT/NL2006/050274
(62) Divisional of application: 07834655.8
(71) Applicant: N.V. Nutricia, 2712 HM Zoetermeer (NL)
(72) Inventor: Zwijsen, Renate Maria Louise, 3634 GK Den Dolder (NL); van de Heijning, Hubertus Josephus Maria, 3523 PN Utrecht (NL); van der Beek, Eline Marleen, 6705 DB Wageningen (NL); van Laere, Katrien Maria Jozefa, 6666 WS Heteren (NL); Boehm, Günther, 61209 Echzell (DE)
(74) Representative: de Boer, Henricus J.R.

(57) **Abstract**

The present invention relates to a method for preventing and/or treating visceral adiposity by administering a certain nutritional composition to an infant with the age between 0 and 36 months, and preventing the occurrence of diseases later in life.

## Description

### FIELD OF THE INVENTION

The present invention relates to preventing diseases later in life by administering particular nutritional composition to infants with the age below 3 years. The present invention relates especially to preventing visceral adiposity.

### BACKGROUND OF THE INVENTION

Breast-feeding is the preferred method of feeding infants. It has been suggested that breast feeding early in life might influence the occurrence of disorders later in life. However, there are circumstances that make breast-feeding impossible or less desirable. In those cases infant formula and follow-on formula are a good alternative. The composition of modem infant or follow-on formulas is adapted in such a way that it meets many of the special nutritional requirements of the fast growing and developing infant.

Still it seems that improvements can be made towards the constitution of infant milk formula. For example little is known about the effects of ingredients in the infant formula on health later in life. The present invention relates to such future health.

WO 2005063050 describes a method of increasing lean body mass and reducing fat body mass in infants by administering to an infant, term or preterm, a nutritional formula comprising a source of DHA and ARA. WO 2006057551 relates to an infant nutrition comprising at least one protease inhibitor, a process for preparing such an infant nutrition and use of the infant nutrition for the treatment and/or prevention of childhood obesity and secondary disorders resulting from childhood obesity. WO 03005836 describes dietary products for infant, child and adult nutrition which possess adequate levels and ratios of medium chain fatty acids and omega-polyunsaturated fatty acids. Consumption of these dietary products can contribute to the prevention of obesity in developing individuals and can contribute to a reduction in body fat mass in individuals who are trying to loose weight or reduce body fat mass (e.g., obese individuals). WO 2006069918 describes a method of continuously reducing the circulating level of insulin like growth factor 1 (IGF-1) in the first few months of the life of an infant by administering to the infant a nutritional composition comprising proteins in an amount such that the composition contains less than 2.25g of protein per 100kcal. As IGF-1 is known to be a key control point in nutritional regulation of growth, this may offer a method of reducing the risk of developing obesity later life. Aillaud et al, 2006, Progress in Lipid research 45:203-236, discusses the role of n6 polyunsaturated fatty acids in the excessive adipose tissue development and relationship to obesity.

### SUMMARY OF THE INVENTION

The present inventors have recognized certain gaps in the present knowledge on the relation between nutrition in early infancy and the development of diseases later in life.

The present inventors have determined that the whole adipose tissue mass of infants is not a good predictor to determine the risks of diseases later in life. Body fat can be distributed and stored in fat tissue at different places within the body. Different fat tissues have different metabolic effects, particularly in infants. Subcutaneous fat in infants has the important function to maintain an adequate body temperature. Fat deposited in the central part of the body (visceral fat) serves only as storage of energy. Moreover, adipose cells at different locations differ in size and in their protein secretion profile which are potential regulators of glucose and lipid homeostasis. Importantly, visceral fat mass is a highly metabolically active tissue that releases free fatty acids directly into the hepatic portal vein. The obtained free fatty acid fluxes have an impact on glucose metabolism and insulin sensitivity of the liver and subsequently can lead to metabolic disorders. Therefore, a main contributor of the development of certain disorders later in life appeared to be the visceral fat mass development in early infancy (visceral adiposity in early infancy). Visceral fat mass accumulation was found to be a main contributor of development disorders, particularly of metabolic and cardiovascular disorders, independent of overall obesity. Moreover, humans may suffer from overall obesity, but not visceral adiposity, whereas visa versa human subjects may suffer from visceral adiposity but not from overall obesity (Lemieux et al, 1996, Am J Clin Nutr, 64:685-693; Carey, 1998, Curr Opinio Lipidology 9:35-40, Matsuzawa et al, 1995, Obesity Research 3 suppl 2:187s194s). Thus, visceral adiposity differs from general obesity not only regarding the health risk on secondary disorders later in life, but also in patient population. Hence, subsequent to this finding it is the aim of the present invention to prevent disorders later in life and reduce visceral fat adipocyte formation in infancy.

It was found that in early infancy, and to a lesser extent during puberty, visceral adipocyte count is determined. In the other stages of life visceral fat mass is "only" increased through filling of the adipocytes with fat. Cell numbers remain approximately the same throughout adult life. Hence, it is highly desirable to reduce visceral adipocyte proliferation in early infancy without excessively reducing subcutaneous fat mass. Reduced visceral adipocyte count prevents disorders later in life.

Finding adequate measures to modify visceral adipocyte proliferation in infants is particularly difficult, as administration of pharmaceutical compounds is generally unacceptable and nutrition cannot be rigorously modified because the infant needs to receive sufficient nutrients for normal growth and development. Furthermore, a-specific reduction of fat mass can be disadvantageous, e.g. because the subcutaneous fat mass has the important role of maintaining body temperature. Hence, a main aim of the present invention is to reduce the development of visceral fat mass accumulation in an infant and/or to reduce the development of visceral fat adipocyte accumulation, preferably later in life by designing a nutrition to be administered to the infant which ensures maintenance of normal growth and development.

The present inventors found a nutritional composition which reduced visceral adiposity and other disorders later in life, particularly diabetes (particularly type 2 diabetes), fasting hyperglycaemia, insulin resistance, hyperinsulinemia, hypertension, cardiovascular disease, cerebrovascular disease, arthrosclerosis, dyslipidaemia, hyperuricaemia, fatty liver, osteoarthritis and sleep apnoea.

The inventors experimentally evidenced that early-in-life administering nutrition wherein the lipid component is relatively low in linoleic acid (LA) and with a low linoleic acid/alpha-linolenic acid (LA/ALA) ratio, results in a decreased visceral fat mass accumulation and/or visceral adiposity, particularly results in a decreased visceral fat mass accumulation and/or visceral adiposity later in life. In the experiments, rodents received a specific nutrition (low LA, low LA/ALA ratio) early-in-life (via the nursing mother), while a control group did not receive the specific nutrition. At the later-in-life stage the animal groups received the same diet high in saturated fat. Surprisingly, not only a decrease in total body fat was observed in rodents which had been fed this experimental nutrition, but also a specific decrease in visceral fat mass, i.e. a decreased visceral adiposity, was observed. This is indicative for the effect of the present composition in the development of diseases later in life, particularly during childhood (age 3-12), adolescence (age 13-18) and adulthood (age above 18).

In a further aspect it was also found that long chain polyunsaturated fatty acids (LC-PUFA), particularly the n3 LC-PUFA docosahexaenoic acid (DHA), stearidonic acid (SDA), docosapentaenoic acid (DPA) and/or eicosapentaenoic acid (EPA), also reduced the visceral fat mass accumulation while maintaining normal growth and development. LC-PUFA are therefore advantageously incorporated in compositions for reducing visceral adiposity and/or disorders later in life. Medium chain fatty acids (MCFA) resulted in an overall reduced fat content later in life. MCFA are therefore advantageously included in the present composition, but in a limited amount. Furthermore, the inclusion of MCFA is particularly desirable in the present low LA formula as it prevents LA deficiency.

The inventors also found that the administration of galacto-oligosaccharides resulted in a decreased insulin peak, while maintaining comparable blood glucose levels. Since high insulin levels stimulate the growth of visceral adipocytes one embodiment is the use of galacto-oligosaccharides for an infant nutrition to prevent visceral adiposity.

### DETAILED DESCRIPTION OF THE INVENTION

In one embodiment the present invention concerns the use of a composition comprising a lipid, protein and digestible carbohydrate component wherein the lipid component provides 35 to 55% of the total calories, the protein component provides 5 to 15% of the total calories and the digestible carbohydrate component provides 30 to 60% of the total calories, wherein the lipid component comprises (i) linoleic acid (LA) and alpha-linolenic acid (ALA) in a weight ratio of LA/ALA between 2 and 6; (ii) less than 14.5 wt.% LA based on total fatty acids; (iii) long chain polyunsaturated fatty acids (LC-PUFA); and optionally (iv) 10-50 wt.% medium chain fatty acids (MCFA) for the manufacture of a nutritional composition to be administered to a non-obese human with the age below 36 months and for preventing the development of a disorder when said human has an age above 36 months, wherein said disorder is selected from the group consisting of type 2 diabetes, fasting hyperglycaemia, insulin resistance, visceral adiposity, hyperinsulinemia, hypertension, cardiovascular disease, cerebrovascular disease, arthrosclerosis, dyslipidaemia, hyperuricaemia, fatty liver, osteoarthritis and sleep apnoea.

In one embodiment the present invention concerns the use of a composition comprising a lipid, protein and digestible carbohydrate component wherein the lipid component provides 35 to 55% of the total calories, the protein component provides 5 to 15% of the total calories and the digestible carbohydrate component provides 30 to 60% of the total calories, wherein the lipid component comprises less than 14.5 wt.% linoleic acid based on total fatty acids and a weight ratio of linoleic acid (LA) to alpha-linolenic acid (ALA) between 2 and 6, for the manufacture of a nutritional composition for a) preventing and/or treating visceral adiposity and/or b) preventing and/or treating the accumulation of visceral fat mass to an excessive amount wherein the nutritional composition is administered to an infant with the age between 0 and 36 months.

The present invention also concerns a composition to be administered to a non-obese human with the age below 36 months, said composition comprising a lipid, protein and digestible carbohydrate component wherein the lipid component provides 35 to 55% of the total calories, the protein component provides 5 to 15% of the total calories and the digestible carbohydrate component provides 30 to 60% of the total calories, wherein the lipid component comprises (i) linoleic acid (LA) and alpha-linolenic acid (ALA) in a weight ratio of LA/ALA between 2 and 6; (ii) less than 14.5 wt.% LA based on total fatty acids; (iii) long chain polyunsaturated fatty acids (LC-PUFA); and optionally (iv) 10-50 wt.% medium chain fatty acids (MCFA) for preventing the development of a disorder when said human has an age above 36 months, wherein said disorder is selected from the group consisting of type 2 diabetes, fasting hyperglycaemia, insulin resistance, visceral adiposity, hyperinsulinemia, hypertension, cardiovascular disease, cerebrovascular disease, arthrosclerosis, dyslipidaemia, hyperuricaemia, fatty liver, osteoarthritis and sleep apnoea.

The present invention also concerns a composition to be administered to an infant with the age between 0 and 36 months, said composition comprising a lipid, protein and digestible carbohydrate component wherein the lipid component provides 35 to 55% of the total calories, the protein component provides 5 to 15% of the total calories and the digestible carbohydrate component provides 30 to 60% of the total calories, wherein the lipid component comprises less than 14.5 wt.% linoleic acid based on total fatty acids and a weight ratio of linoleic acid (LA) to alpha-linolenic acid (ALA) between 2 and 6, for a) preventing and/or treating visceral adiposity and/or b) preventing and/or treating the accumulation of visceral fat mass to an excessive amount.

In one embodiment of the present invention concerns the use of a composition comprising a galactooligosaccharides and/or long chain polyunsaturated fatty acid for the manufacture of a nutritional composition to be administered to a non-obese human with the age below 36 months for a) preventing and/or treating visceral adiposity and/or b) preventing and/or treating the accumulation of visceral fat mass to an excessive amount.

### Visceral adiposity

The term 'visceral adiposity' refers to a condition with increased visceral fat mass. The term visceral adiposity is also referred to as central obesity. Visceral adiposity is typically caused by (accumulation of) excessive visceral fat mass. Visceral fat, also known as organ fat, intra-abdominal fat, peritoneal fat or central fat is normally located inside the peritoneal cavity as opposed to subcutaneous fat which is found underneath the skin and intramuscular fat which is found interspersed in skeletal muscles. Visceral fat includes mesenteric fat, perirenal fat and retroperitoneal fat. Visceral fat stores can suitably be investigated by imaging techniques such as computed tomography (CT), magnetic resonance imaging (MRI) and ultrasonography. An adult is considered to suffer from visceral adiposity or to have accumulated visceral fat mass to an excessive amount when at the umbilicus level the visceral adipose tissue (VAT) exceeds 100 cm² in man, or 90 cm² in women (Saito et al, 1009, Int J Obes Suppl 3:S226). Infants generally have about 10% visceral fat mass based on total fat mass. The term visceral adiposity in infants therefore preferably relates to a situation wherein the visceral fat mass exceeds about 10 wt.% based on total fat (e.g. between 10 and 20 wt.% visceral fat mass based on total fat mass). In humans beyond infancy, particularly of or above the age of 12 years, visceral adiposity is also referred to as 'apple-shaped' obesity, 'android' obesity, 'abdominal' obesity, 'male-type' obesity, 'waist-predominant' obesity, 'truncal' obesity or 'masculine' obesity. A waist circumference above 102 cm in adult man or above 88 cm in adult women indicates visceral adiposity. Also a waist-hip ratio can be used as indicator for visceral adiposity. Hip-waist ratio's exceeding 0.9 in man and 0.85 in women indicate visceral adiposity. For children of 3-19 years old appropriate cutoffs for age- and sex-dependent waist circumferences can be found in Taylor et al, 2000 Am J Clin Nutr 72:490-495. A subject suffers from visceral adiposity when it meets one or more of the above criteria (regarding VAT, waist circumference or waist-hip ratio treshholds). Accumulation of visceral fat mass to an excessive amount relates to the accumulation of visceral fat mass to a level at which visceral adiposity occurs and can be determined by the same methods as described above for visceral adiposity.
Total fat mass can be determined by DEXA (dual-energy X-ray absorptiometry). The present composition is preferably administered to a non-obese human with the age below 36 months, preferably to a non-overweight human with the age below 36 months. Obesity and/or overweight can suitably be determined by a physician. Typically, a non-obese infant below 36 months of age has gender specific weight-for -length below the 95^{th} percentile, more preferably below the 85^{th} percentile. Gender specific weight-for-length percentiles have been published by Center for Disease Control and Prevention (CDC) in 2000.

### Lipid component

Herein LA refers to linoleic acid (18:2 n6); ALA refers to alpha-linolenic acid (18:3 n3); LC-PUFA refers to long chain polyunsaturated fatty acids comprising at least 20 carbon atoms in the fatty acid chain and with 2 or more unsaturated bonds; DHA refers to docosahexaenoic acid (22:6, n3); EPA refers to eicosapentaenoic acid (20:5 n3); ARA refers to arachidonic acid (20:4 n6); Medium chain fatty acids (MCFA) are fatty acids and/or acyl chains with a chain length of 6, 8 or 10 carbon atoms. MCFA may also be referred to as medium chain triglycerides (MCT).

The present inventors have found that specific compositions that have a low LA/ALA ratio and that are low in LA and that optionally comprise LC-PUFA and optionally comprise MCFA prevent the occurrence of visceral adiposity. Particularly the administration of a nutritional composition comprising (i) a LA/ALA ratio between 2 and 6 and (ii) a low LA content (<14.5 wt.% based on total fatty acids) and optionally LC-PUFA (particularly DHA) resulted in a decrease in visceral adiposity later in life and/or a reduced occurrence of disorders later in life. MCFA were found to reduce adiposity in general. This finding further enables the development of an optimal composition, which comprises MCFA, but not in excessive amounts, i.e. between 10 and 50 wt.% based on total weight of fatty acids.

The present composition comprises lipid. LA should be present in a sufficient amount in order to promote a healthy growth and development, yet in an amount as low as possible to prevent occurrence of visceral adiposity. The composition therefore comprises less than 14.5 wt.% LA based on total fatty acids, preferably between 5 and 14.5 wt.%, more preferably between 6 and 12 wt.%. Based on total dry weight of the composition the present composition preferably comprises 2 to 5 wt.% LA. When in liquid form, e.g. as ready-to-drink formula, the LA content is preferably between 0.3 and 0.55 g LA per 100 ml of the liquid composition. The LA preferably provides between 4 to 8% of total calories in the present composition.

ALA should be present in a sufficient amount to promote a healthy growth and development of the infant. The present composition therefore preferably comprises at least 1.6 wt.% ALA based on total fatty acids, preferably between 1.6 and 5 wt.% ALA. Based on total dry weight of the composition the present composition preferably comprises at least 0.30 wt.% ALA, preferably between 0.3 and 1 wt.% ALA. When in liquid form, e.g. as ready-to-drink formula, the ALA content is preferably at least 50 mg ALA per 100 ml of the liquid composition, preferably between 50 and 150 mg ALA per 100 ml.

The weight ratio LA/ALA should be well balanced in order to prevent visceral fat mass deposition (e.g. visceral adiposity) and disorders later in life, while at the same time ensure a normal growth and development. The proper ratio was found by the present inventors. The present composition comprises a weight ratio of LA/ALA between 2 and 6, more preferably between 3 and 6, even more preferably between 4 and 5.5, even more preferably between 4 and 5. The lipid component comprises less than 14.5 wt.% LA based on total fatty acids and a LA/ALA ratio of 2 to 6.

Preferably the present composition comprises LC-PUFA. The present inventors found that LC-PUFA reduces visceral adiposity later in life. More preferably, the present composition comprises n-3 LC-PUFA, even more preferably EPA, SDA, DPA and/or DHA. It was found that these LC-PUFA decrease the visceral adiposity.
Since a low concentration of DHA, SDA, DPA and/or EPA is already effective and normal growth and development are important, the content of LC-PUFA in the present composition, preferably does not exceed 15 wt.% of the total fat content, preferably does not exceed 10 wt.%, even more preferably does not exceed 5 wt.%. Preferably the present composition comprises at least 0.1 wt.%, preferably at least 0.25 wt.%, more preferably at least 0.5 wt.%, even more preferably at least 0.75 wt.% LC-PUFA of the total fat content. For the same reason, the EPA content preferably does not exceed 5 wt.% of the total fat, more preferably does not exceed 1 wt.%, but is preferably at least 0.025 wt.%, more preferably at least 0.05 wt.% of the total fat. The DHA content preferably does not exceed 5 wt.%, more preferably does not exceed 1 wt.%, but is at least 0.1 wt.% of the total fat. The DPA content preferably does not exceed 1 wt.%, more preferably does not exceed 0.5 wt.% of the total fat, but is preferably at least 0.01 wt.% of the total fat. The SDA content preferably does not exceed 0.5 wt.%, more preferably does not exceed 0.25 wt.% of the total fat, but is at least 0.005 wt.% of the total fat.

As arachidonic acid (ARA, an n6 PUFA) counteracts the effect, the present composition comprises relatively low amounts or ARA. The ARA content preferably does not exceed 5 wt.%, more preferably does not exceed 1 wt.%, more preferably; does not exceed 0.5 wt.%, even more preferably does not exceed 0.25 wt.%, most preferably does not exceed 0.05 wt.% based on total fatty acids.
However, ARA plays an important role in neurological development in infants. Since the amount of LA (an n6 fatty acid) is preferably low and the conversion of LA to ARA is in infants less efficient, preferably at least 0.02 wt.%, more preferably at least 0.05 wt.%, more preferably; at least 0.1 wt.%, even more preferably at least 0.2 wt.%, based on total fatty acids.

The LC-PUFA, LA and/or ALA may be provided as free fatty acids, in triglyceride form, in diglyceride form, in monoglyceride form, in phospholipid form, or as a mixture of one of more of the above. Preferably the present composition contains LC-PUFA in triglyceride and/or phospholipid form.

Medium chain fatty acids (MCFA) are fatty acids and/or acyl chains with a chain length of 6, 8 or 10 carbon atoms. The present low LA and low LA/ALA composition advantageously comprises 10-50 wt.% MCFA based on total fatty acids. LA is an essential fatty acid, meaning that it cannot be synthesized within the body. As the present composition comprises a relatively low LA content, it is important that the LA included in the present composition is not converted to energy (by fat oxidation) and therefore not available for anabolic purposes. To reduce the oxidation of LA in the present low LA composition MCFA can suitably be added. MCFA are easily mobilized in the bloodstream to provide long-lasting energy, rather than being stored as fat and thereby reduce LA oxidation.
The present inventors also found that MCFA contribute to a reduced fat mass later in life. Therefore the present composition advantageously comprises MCFA. However, the MCFA reduce the accumulation of fat but were found to have less specific effect on visceral fat mass reduction compared to the effect of low LA and low LA/ALA. Therefore, the present composition preferably comprises 10 to 50 wt.% MCFA based on total fatty acids, more preferably 20 to 40 wt.%.

Preferably the present composition contains at least one, preferably at least two lipid sources selected from the group consisting of linseed oil (flaxseed oil), rape seed oil (including colza oil, low erucic acid rape seed oil and canola oil), salvia oil, perilla oil, purslane oil, lingonberry oil, sea buckthorn oil, hemp oil, high oleic sunflower oil, high oleic safflower oil, olive oil, marine oils, microbial oils, black currant seed oil, echium oil, butter fat, coconut oil and palm kernel oil. Preferably the present composition contains at least one, preferably at least two lipid sources are selected from the group consisting of linseed oil, rapeseed oil, coconut oil, high oleic sunflower oil, butter oil and marine oil.

Table 1 gives preferred characteristics of the lipid component of the present composition

**TABLE 1**

| | preferred | more preferred | most preferred |
|---|---|---|---|
| LA (wt.% based on total fatty acids) | <14.5 | 5-14.5 | 6-12 |
| Weight ratio LA/ALA | 2-6 | 3-6 | 4-5.5 |
| MCFA (wt.% based on total fatty acids) | 10-50 | 10-40 | 15-25 |
| n3 LC-PUFA (wt.% based on total fatty acids) | 0.1-15 | 0.25-10 | 0.5-5 |

### Non-digestible oligosaccharides

One effect of the visceral fat mass is believed to be the stimulation of insulin production, with can result over time in a reduced insulin sensitivity and other disorders and other metabolic disorders. High blood insulin levels stimulate glucose uptake in adipose tissue, resulting in an increased adipose tissue mass. In infants the high insulin levels contributes to increased proliferation of visceral adipocytes, at least partly due to the increased glucose uptake.
The present composition therefore preferably maintains low insulin levels. The present composition preferably comprises an ingredient which increases and/or maintains insulin sensitivity. It was found that non-digestible oligosaccharides (NDO) that can be fermented (particularly galacto-oligosaccharides) have a blood insulin tempering effect, and consequently contributes to a reduced proliferation of visceral adipocytes.
Additionally it was also recognised that infants ingest more calories when bottle fed compared to a situation where breast feeding occurs. In addition to the compositional features of the lipid component as suggested in the present invention, the effectiveness can further be improved by reducing caloric intake. Limiting dosage of the nutritional composition is however not a feasible option for infants. The present inventors have found that advantageously the present composition comprises non-digestible oligosaccharides.

Therefore, the present composition preferably comprises the present lipid component and a non-digestible oligosaccharide which can be fermented. The combination of the present lipid component and the non-digestible oligosaccharides synergistically reduces the visceral adiposity and/or prevents the development of disorders later in life. Preferably the non-digestible oligosaccharides have a DP between 2 and 60. The composition preferably prevents the onset of insulin resistance. The non-digestible oligosaccharide is preferably selected from the group consisting of fructo-oligosaccharides (including inulin), galacto-oligosaccharides (including transgalacto-oligosaccharides), gluco-oligosaccharides (including gentio-, nigero- and cyclodextrin-oligosaccharides), arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides. Preferably the present composition comprises fructo-oligosaccharides, galacto-oligosaccharides and/or galacturonic acid oligosaccharides, more preferably galacto-oligosaccharides, most preferably transgalacto-oligosaccharides. In a preferred embodiment the composition comprises a mixture of transgalacto-oligosaccharides and fructo-oligosaccharides. Preferably the present composition comprises galacto-oligosaccharides with a DP of 2-10 and/or fructooligosaccharides with a DP of 2-60. The galacto-oligosaccharide is preferably selected from the group consisting of transgalacto-oligosaccharides, lacto-N-tetraose (LNT), lacto-N-neotetraose (neo-LNT), fucosyl-lactose, fucosylated LNT and fucosylated neo-LNT. In a particularly preferred embodiment the present method comprises the administration of transgalacto-oligosaccharides ([galactose]ₙ-glucose; wherein n is an integer between 1 and 60, i.e. 2, 3, 4, 5, 6, ...., 59 ,60; preferably n is selected from 2, 3, 4, 5, 6, 7, 8, 9, or 10). Transgalacto-oligosaccharides (TOS) are for example sold under the trademark Vivinal™ (Borculo Domo Ingredients, Netherlands). Preferably the saccharides of the transgalacto-oligosaccharides are β-linked. Fructo-oligosaccharide is a NDO comprising a chain of β linked fructose units with a DP or average DP of 2 to 250, more preferably 10 to 100. Fructo-oligosaccharide includes inulin, levan and/or a mixed type of polyfructan. An especially preferred fructo-oligosaccharide is inulin. Fructo-oligosaccharide suitable for use in the compositions is also already commercially available, e.g. Raftiline®HP (Orafti). Uronic acid oligosaccharides are preferably obtained from pectin degradation. Hence the present composition preferably comprises a pectin degradation product with a DP between 2 and 100. Preferably the pectin degradation products is prepared from apple pectin, beet pectin and/or citrus pectin. Preferably the composition comprises transgalacto-oligosaccharide, fructo-oligosaccharide and pectin degradation product. The weight ratio transgalacto-oligosaccharide : fructo-oligosaccharide : pectin degradation product is preferably 20-2 : 1 : 1-3, more preferably 12-7 : 1 : 1-2.
Preferably, the composition comprises of 80 mg to 2 g non-digestible oligosaccharides per 100 ml, more preferably 150 mg to 1.50 g, even more preferably 300 mg to 1 g per 100 ml. Based on dry weight, the composition preferably comprises 0.25 wt.% to 5.5 wt.%, more preferably 0.5 wt.% to 4 wt.%, even more preferably 1.5 wt.% to 3 wt.% non-digestible oligosaccharides.

### Lactose

The maintenance of insulin sensitivity can be further improved by inclusion of a low glycaemic carbohydrate in the present composition, preferably lactose. Hence, the present composition preferably comprises in addition to the present lipid component, non-digestible oligosaccharides and/or lactose. The present composition preferably comprises a digestible carbohydrate component, wherein at least 35 wt.%, more preferably at least 50 wt.%, more preferably at least 75 wt.%, even more preferably at least 90 wt.%, most preferably at least 95 wt.% is lactose. The present composition preferably comprises at least 25 grams lactose per 100 gram dry weight of the present composition, preferably at least 40 grams lactose/100 gram.

### Hydrolyzed protein

Preferably the composition comprises hydrolyzed casein and/or hydrolyzed whey protein. It was found that administration of a composition wherein the protein comprises hydrolyzed casein and hydrolyzed whey results in reduced post-prandial levels of both insulin and glucose compared to the administration of a composition comprising intact casein and intact whey protein. Increased levels of both insulin and glucose indicate a form of insulin resistance in formula fed infants, which is believed contribute to the development of visceral adiposity later-in-life. The present composition preferably comprises at least 25 wt.% peptides with a chain length of 2 to 30 amino acids based on dry weight of protein. The amount of peptides with a chain length between 2 and 30 amino acids can for example be determined as described by de Freitas et al, 1993, J. Agric. Food Chem. 41:1432-1438. The present composition preferably comprises casein hydrolysate and/or whey protein hydrolysate, more preferably casein hydrolysate and whey protein hydrolysate because the amino acid composition of bovine casein is more similar to the amino acid composition found in human milk protein and whey protein is easier to digest and found in greater ratios in human milk. The composition preferably comprises at least 50 wt.%, preferably at least 80 wt.%, most preferably about 100 wt.% of a protein hydrolysate, based on total weight of the protein. The present composition preferably comprises a protein with a degree of hydrolysis of the protein between 5 and 25%, more preferably between 7.5 and 21%, most preferably between 10 and 20%. The degree of hydrolysis is defined as the percentage of peptide bonds which have been broken down by enzymatic hydrolysis, with 100% being the total potential peptide bonds present. The present composition preferably contains 1.5 to 2.25 g protein/100 kcal, preferably between and 1.8 and 2.0 g/100 kcal.

### Casein

Casein is advantageously present since it increases the gastric emptying times by forming a curd in the stomach, thereby increasing satiety. As satiety induction is highly desirable, see above, the present composition preferably comprises casein. When the composition is in liquid form, e.g. as a ready-to-drink liquid, the composition preferably comprises at least 0.5 g casein per 100 ml, preferably between 0.5 and 5 gram casein per 100 ml. Preferably the composition comprises at least 4 wt.% casein based on dry weight. Preferably the casein is intact and/or non-hydrolyzed.

### Nutritional composition

The present composition is particularly suitable for providing the daily nutritional requirements to an infant with the age below 36 months, particularly an infant with the age below 24 months, even more preferably an infant with the age below 12 months. Hence, the present composition comprises a lipid, protein and digestible carbohydrate component wherein the lipid component provides 35 to 55% of the total calories, the protein component provides 5 to 15% of the total calories and the digestible carbohydrate component provides 30 to 60% of the total calories. Preferably the present composition comprises a lipid component providing 40 to 50 % of the total calories, the protein component provides 6 to 12% of the total calories and the digestible carbohydrate component provides 40 to 50% of the total calories. When in liquid form, e.g. as a ready-to-feed liquid, the composition preferably comprises 2.1 to 6.5 g fat per 100 ml, more preferably 3.0 to 4.0 g per 100 ml. Based on dry weight the present composition preferably comprises 12.5 to 40 wt.% fat, more preferably 19 to 30 wt.%.

The amount of saturated fatty acids is preferably below 58 wt.% based on total fatty acids, more preferably below 45 wt.%. The concentration of monounsaturated fatty acids preferably ranges from 17 to 60% based on weight of total fatty acids.
The present composition is not human breast milk. The present composition preferably comprises (i) vegetable lipid and/or animal (non-human) fat; and/or (ii) vegetable protein and/or animal (non-human) milk protein. Examples of animal milk protein are whey protein from cow's milk and protein from goat milk. Preferably the present composition does not comprise a proteinase inhibitor, preferably not a trypsin inhibitor, chymotrypsin inhibitor or elastase inhibitor.

The present composition preferably comprises at least 50 wt.% protein derived from non-human milk based on total protein, more preferably at least 90 wt.%. Preferably the present composition comprises at least 50 wt.% cow milk derived protein based on total protein, more preferably at least 90 wt.%. Preferably the present composition comprises acid whey and/or sweet whey with a reduced concentration of glycomacropeptide. Preferably the present composition comprises protein derived from β-casein and/or α-lactalbumin. The present composition preferably comprises casein and whey proteins in a weight ratio casein:whey of 10:90 to 90:10, more preferably 20:80 to 80:20. The term protein as used in the present invention refers to the sum of proteins, peptides and free amino acids.

The present composition is preferably administered in liquid form. In order to meet the caloric requirements of the infant, the composition preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the present composition is preferably between 150 and 420 mOsmol/1, more preferably 260 to 320 mOsmol/l. The low osmolarity aims to reduce the gastrointestinal stress. Stress can induce adiposite formation.
Preferably the composition is in a liquid form, with a viscosity below 35 cps as measured in a Brookfield viscometer at 20°C at a shear rate of 100 s⁻¹. Suitably, the composition is in a powdered from, which can be reconstituted with water to form a liquid, or in a liquid concentrate form, which should be diluted with water.
When the composition is a liquid form, the preferred volume administered on a daily basis is in the range of about 80 to 2500 ml, more preferably about 450 to 1000 ml per day.

### Infant

Visceral adipocytes proliferate during the first 36 months of life as well as (more limited) in puberty. Hence the present composition is administered to the infant during the first 3 years of life. It was found that there is a predominance of proliferation of visceral adipocytes in the first 12 months of life (optimum in perinatal adipocyte proliferation). Hence, it is particularly important that the present composition is administered to the infant in this period of life. The present composition is therefore advantageously administered to a human of 0-24 months, more preferably to a human of 0-18 months, most preferably to a human of 0-12 months.
In a preferred embodiment the present composition is administered to a preterm infant, in the form of a complete formula and/or in the form of a breast milk fortifier.
The present invention particularly aims to prevent disease development later in life and is preferably not a curative treatment. Hence, the present composition is preferably administered to an infant not suffering from obesity or childhood obesity, particularly a non-obese infant more preferably an infant that does not suffer from overweight. The present composition is preferably administered orally to the infant.

### Application

The composition aims to a) prevent and/or treat visceral adiposity and/or b) prevent and/or treat the accumulation of visceral fat mass to an excessive amount. Particularly, the present invention relates to a method for preventing visceral adiposity in a human with the age below 36 months. The present invention also aims to prevent visceral adiposity at the age above 36 months, particularly to prevent visceral adiposity at the age above 8 years, particularly above 15 years. The present inventors found that the present reduction in visceral adiposity suitably reduces occurrence and prevalence of disorders later in life, particularly disorders linked to visceral adiposity. The present invention also provides a method for preventing the subsequent development of a disorder in a human with an age above 36 months, wherein said disorder is selected from the group consisting of diabetes (particularly type 2 diabetes), fasting hyperglycaemia, insulin resistance, hyperinsulinemia, hypertension, cardiovascular disease, visceral adiposity, cerebrovascular disease, arthrosclerosis, dyslipidaemia, hyperuricaemia, fatty liver, osteoarthritis and sleep apnoea, via the prevention of visceral adiposity and wherein the method comprises the administration of the present composition to an infant with an age below 36 months.
The present invention also provides a method for preventing the development of a disorder in a human with an age above 36 months, wherein said disorder is selected from the group consisting of diabetes (particularly type 2 diabetes), fasting hyperglycaemia, insulin resistance, hyperinsulinemia, hypertension, cardiovascular disease, visceral adiposity, cerebrovascular disease, arthrosclerosis, dyslipidaemia, hyperuricaemia, fatty liver, osteoarthritis and sleep apnoea, wherein the method comprises the administration of the present composition to an infant with an age below 36 months. The term dyslipidaemia includes the following diseases: hyperlipidaemia, hyperlipoproteinaemia, hyperchylomicronaemia, hyper-cholesteraemia, hypoalphalipoproteinemia hypoHDL/LDL-aemia and hyper-triglyceridaemia. Particularly the development of diabetes, visceral adipocity and/or cardiovascular diseases can be prevented, more in particular cardiovascular diseases. The present method is particularly suitable to prevent the abovementioned disorders during adolescence, in particular from 13 - 18 years and/or adulthood, in particular above 18 years.

### Galactooligosaccharides and/or LC-PUFA

The present prevention provides the use of a composition comprising a galactooligosaccharides and/or long chain polyunsaturated fatty acid (LC-PUFA) for the manufacture of a nutritional composition to be administered to a non-obese human with the age below 36 months and for preventing the development of a disorder when said human has an age above 36 months, wherein said disorder is selected from the group consisting of type 2 diabetes, fasting hyperglycaemia, insulin resistance, visceral adiposity, hyperinsulinemia, hypertension, cardiovascular disease, cerebrovascular disease, arthrosclerosis, dyslipidaemia, hyperuricaemia, fatty liver, osteoarthritis and sleep apnoea. The galacto-oligosaccharide is preferably selected from the group consisting of transgalacto-oligosaccharides, lacto-N-tetraose (LNT), lacto-N-neotetraose (neo-LNT), fucosyl-lactose, fucosylated LNT and fucosylated neo-LNT. The LC-PUFA is preferably DHA and/or EPA, preferably DHA and EPA. The nutritional composition is preferably orally administered in the form of a complete nutritional formula. Hence, the nutritional composition preferably comprises a lipid, protein and digestible carbohydrate component wherein the lipid component provides 35 to 55% of the total calories, the protein component provides 5 to 15% of the total calories and the digestible carbohydrate component provides 30 to 60% of the total calories. Preferred amounts of galactooligosaccharides and/or LC-PUFA are described hereinabove.
Preferably the composition comprises both galacto-oligosaccharides and LC-PUFA.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Programming effect of dietary fat on adult fat tissue

Offspring of C57/BL6 dams was standardized on postnatal day 2 to nests of 6 pups (4M and 2F) per dam. Dams were fed the experimental diet from day 2 onward until weaning. The lipid composition of the mouse milk reflects the fat composition of the diet. After weaning the male mice were housed in pairs and the experimental diet was continued until day 42 when all pups were fed the same diet containing lard and extra cholesterol. The experimental diets that were used were: 1) LC-PUFA diet (tuna fish

**Table 2: Fatty acid composition of the diets**

| | | control | MCFA | LC-PUFA | Low LA | Cafetaria |
|---|---|---|---|---|---|---|
| | | g/100g fat | g/100g fat | g/100g fat | g/100g fat | g/100g fat |
| C-4:0 | | 0.00 | 0.00 | 0.00 | 1.05 | 0.00 |
| C-6:0 | | 0.11 | 0.15 | 0.07 | 0.81 | 0.06 |
| C-8:0 | | 1.70 | 11.42 | 1.07 | 2.09 | 0.85 |
| C-10:0 | | 1.36 | 8.77 | 0.86 | 2.17 | 0.68 |
| C-12:0 | | 10.53 | 1.34 | 6.69 | 11.42 | 5.27 |
| C-14:0 | | 4.38 | 0.75 | 3.62 | 7.24 | 2.69 |
| C-14:1w5 | | 0.00 | 0.00 | 0.00 | 0.00 | 0.00 |
| C-15:0 | | 0.00 | 0.00 | 0.00 | 0.00 | 0.00 |
| C-16:0 | | 17.14 | 13.35 | 19.38 | 12.40 | 23.07 |
| C-16:1w7 | | 0.13 | 0.12 | 1.20 | 0.78 | 1.56 |
| C-17:0 | | 0.00 | 0.00 | 0.37 | 0.00 | 0.00 |
| C-18:0 | | 3.07 | 2.39 | 3.70 | 5.12 | 9.03 |
| C-18:1w9 | | 37.94 | 38.52 | 35.27 | 40.79 | 40.47 |
| C-18:2w6 | LA | 14.80 | 14.31 | 11.89 | 6.38 | 11.90 |
| C-18:3w3 | ALA | 2.61 | 2.61 | 1.07 | 1.57 | 1.30 |
| C-18:3w6 | | 0.00 | 0.00 | 0.00 | 0.00 | 0.00 |
| C-18:4w3 | SDA | 0.00 | 0.00 | 0.19 | 0.00 | 0.00 |
| C-20:0 | | 0.34 | 0.34 | 0.26 | 0.20 | 0.17 |
| C-20:1w9 | | 0.41 | 0.41 | 0.15 | 0.22 | 0.21 |
| C-20:2w6 | | 0.00 | 0.00 | 0.00 | 0.00 | 0.00 |
| C-20:3w6 | | 0.00 | 0.00 | 0.00 | 0.00 | 0.00 |
| C-20:4w3 | | 0.00 | 0.00 | 0.07 | 0.00 | 0.00 |
| C-20:4w6 | AA | 0.00 | 0.00 | 0.28 | 0.00 | 0.00 |
| C-20:5w3 | EPA | 0.00 | 0.00 | 1.20 | 0.00 | 0.00 |
| C-22:0 | | 0.23 | 0.28 | 0.24 | 0.33 | 0.11 |
| C-22:1w9 | | 0.14 | 0.14 | 0.05 | 0.08 | 0.07 |
| C-22:4w6 | | 0.00 | 0.00 | 0.00 | 0.00 | 0.00 |
| C-22:5w3 | DPA | 0.00 | 0.00 | 0.37 | 0.00 | 0.00 |
| C-22:6w3 | DHA | 0.00 | 0.00 | 5.00 | 0.00 | 0.00 |
| C-24:0 | | 0.02 | 0.02 | 0.02 | 0.00 | 0.01 |
| C-24:1w9 | | 0.00 | 0.00 | 0.00 | 0.00 | 0.00 |
| cholesterol | | | | | | 0.10 |
| | total | 94.91 | 94.92 | 93.02 | 92.66 | 98.46 |

oil); 2) Low LA diet (butter oil; low in canola oil, high in Trisun 80, no palm oil); 3) MCFA diet; 4) control diet (similar amounts of canola oil, coconut oil and palm oil). The fatty acid composition of the diets is presented in Table 2. At day 42, all mice switched to a "cafeteria diet" comprising 10 wt.% fat (3 to 5 wt.% lard fat and 0.1 wt.% cholesterol) until day 98. The mice were weighed twice a week. The food intake was determined once a week during the entire experiment. To determine body composition (i.e., fat mass (FM) and fat-free mass (FFM)) DEXA scans (Dual Energy X-ray Absorbiometry) were performed under general anesthesia at 6, 10 and 14 weeks of age, 42, 70 and 98 days after birth respectively, by densitometry using a PIXImus imager (GE Lunar, Madison, WI, USA). At the age of 14 weeks the male mice were sacrified and plasma, epididymal fat, perirenal fat, pancreas, liver and kidneys were dissected and weighed.

Results: No effect on growth (body weight) and food intake was observed during the experimental period between the groups (data not shown). Moreover, the development of fat mass (determined with DEXA) was slightly lower in the MCFA, LC-PUFA and low LA groups compared to control but not significantly different at day 42 (end of the diet intervention period). However, a subsequent treatment with a cafetaria diet (high in saturated fatty acids) between day 42 and day 98 of all groups resulted in clear differences in body composition at the end of the experiment (day 98), see Table 3. The fat mass was reduced when the pups received a LC-PUFA, MCFA or low LA diet in their early life, compared to the control diet. Moreover, the diets in the early life had markedly effect on the body fat distribution. It was shown that the ratio of the subcutaneous:visceral fat (measured by epididymal and perirenal fat, respectively) in adult mice at day 98 was increased by 14% in the LC-PUFA group and 32% in the low LA group, but was not increased in the MCFA group compared to the control group, see Table 3.

**Table 3: Fat % development of total body mass in time, absolute fat weight and ratio's of subcutaneous (epididymal) and central (perirenal) fat at day 98.**

| | Day | Control diet | MCFA diet | LC-PUFA diet | Low LA diet |
|---|---|---|---|---|---|
| Fat % | 42 | 19.6 | 17.1 | 16.6 | 17.1 |
| Fat % | 70 | 22.1 | 22.8 | 21.5 | 24.2 |
| Fat % | 98 | 26.9 | 22.8 | 20.9 | 24.2 |
| Visceral fat (mg) | 98 | 89 | 76 | 71 | 72 |
| % visceral fat decrease | 98 | 0 | 14% | 20% | 19% |
| Ratio sub-cutaneous fat/ visceral fat | 98 | 7.54 | 8.35 | 8.59 | 9.92 |
| Ratio increase (%) | | | +11% | +14% | +32% |

Furthermore, the epididymal adipocytes were found to be many, and large and filled with large amounts of fat (hyperplasia) in mice fed with the cafeteria diet, were found to be fewer, but large and filled with large amounts of fat in mice fed the low LA diet, whereas the MCFA diet and the LC PUFA diet resulted in many, smaller cells. This suggests a different mode of action of low LA and LC-PUFA regarding adiposity. Basal insulin levels were significantly lower at day 96 following the MCFA, LC-PUFA and low LA programming diet compared to control, whereas plasma glucose levels were similar. This is indicative for a decreased insulin resistance, according to the homeostatic model assessment (HOMA) index.
This demonstrated that the visceral fat mass in later life clearly is decreased by an early in life diet high in LC-PUFA and/or low in LA and/or low LA/ALA. So, it is concluded that these fat compositions program and/or imprint the body to get a healthier body fat composition later in life.

### Example 2: Blood glucose/insulin and non-digestible oligosaccharides

Animals and treatment: Adult male Wistar rats (n=7) were given a GOS fiber load, cellulose load or water via a gastric canula on day 1. A 6 mL bolus load was administered equal to 50% of their daily fiber intake; GOS fiber used was transgalacto-oligosaccharides obtained from Elix'or (Borculo Domo). Fiber was dissolved in water. About 24 h later (on day 2) an oral glucose tolerance test was carried out and the postprandial glucose and insulin course was monitored for 120 min upon the intragastric injection of a carbohydrate load (2 g/kg body weight). To this end blood samples were drawn repeatedly via a jugular vein canula. Intragastric injection of water or a cellulose solution in water on day 1 served as control. As the GOS fiber preparation consisted of 50% of digestible carbohydrates (mainly lactose), the two control injections were co-administered with carbohydrates to correct for this.

Results: pre-treatment with GOS fibers clearly decreased the amount of insulin secreted, resulting in significant (p<0.05) lower incremental AUC values. Blood glucose levels were not affected significantly. Pre-treatment with cellulose or water did not modulate the insulin secretion, see Table 4.

**Table 4: Insulin and glucose levels levels in rats.**

| Pre-treatment with: | AUC insulin (pM*30 min) | AUC glucose (mM*30 min) |
|---|---|---|
| Water | 41 ± 7 | 69±10 |
| Cellulose | 46 ± 8 | 75 ± 9 |
| GOS | 22 ± 4 | 74±15 |

### Example 3: Hydro lyzed proteins beneficially affect insulin sensitivity

Protein preparations: Intact whey protein, Deminal 90, and skimmed milk powder, were mixed to a 40 wt.% casein and 60 wt.% whey protein ratio. Hydrolyzed whey protein was obtained by hydrolysis of an acid whey preparation as described in examples 1-4 of WO 0141581. The degree of hydrolysis was 15%. Hydrolyzed casein was commercially obtained as LacProdan DI-2038 (Arla Foods). The two preparations were combined at a ratio of 40 wt.% hydrolyzed casein and 60 wt.% hydro lyzed whey.

Methods: 20 adult male Wistar rats (aged 10 weeks at the start of the experiment) were housed individually. After a 4 h fasting period, 10 animals were fed 2 ml of a composition. Three different compositions were tested in a cross-over design (experiments separated by one week). I) human breast milk, ii) 17 mg whey/casein protein and 86 mg lactose per ml. iii) 17 mg hydrolyzed whey and hydrolyzed casein and 86 mg lactose per ml. Subsequently, blood samples (200 µl) were collected in heparinised chilled tubes at t=0, 5, 10, 15, 30, 60, 90, and 120 minutes after feeding. Subsequently, plasma was separated after centrifugation (10 min, 5000 rpm) and stored at -20 °C until analysis. Plasma insulin was measured by radioimmunoassay (RIA, of Linco Research) according to the manufacturer's instructions with the following adjustment: all assay volumes were reduced four times. Plasma glucose was measured with an oxidase-peroxidase method in 96-wells format (Roche Diagnostics, #1448668).

Results: The post-prandial peak of glucose as well as of insulin was lower in rats fed intact whey and intact casein than in rats fed hydrolyzed whey and hydrolyzed casein. The area under the curve (AUC) of insulin and glucose is lower in rats fed hydrolyzed whey and hydrolyzed casein than in rats fed intact whey and intact casein. Also the peak time, maximal peak height, and AUC was lowered when hydrolyzed proteins were consumed (Table 5). The presence of a hydrolyzed proteins resulted in post-prandial blood glucose as well as insulin levels and kinetics more similar to those observed with human milk. Decreased levels of both insulin and glucose indicate increased insulin sensitivity, which is believed contribute to the prevention of central obesity later-in-life.

**Table 5: Effects of intact and hydrolyzed proteins on post-prandial peak time, maximal peak height and area under the curve of glucose and insulin.**

| Effect | | Intact proteins | Hydrolysed proteins | Human milk |
|---|---|---|---|---|
| Peak time (m ± se) | | | | |
| | Glucose | 20.0 ± 12.7 | 11.50 ± 2.6 | 12.0 ± 2.4 |
| | Insulin | 8.3 ± 0.8 | 8.33 ± 1.4 | 11.7 ± 1.2 |
| Maximal peak height (g/l ± se) | | | | |
| | Glucose | 0.46 ± 1.0 | 0.31 ± 0.07 | 0.33 ± 0.08 |
| | Insulin | 1.51 ± 0.44 | 1.11 ± 0.20 | 1.41 ± 0.27 |
| AUC 30 ( ± se) | | | | |
| | Glucose (mM*30min) | 5.9 ± 1.6 | 5.08 ± 1.4 | 5.1 ± 1.8 |
| | Insulin (pM*30min) | 14.3 ± 3.4 | 12.63 ± 4.6 | 11.7 ± 4.7 |

### Example 4: Infant nutrition

Infant nutrition comprising a lipid component providing 48% of the total calories, a protein component providing 8% of the total calories and a digestible carbohydrate component providing 44% of the total calories; (i) the lipid component comprising based on total fatty acids: 10 wt.% LA; 20 wt.% MCFA; 0.2 wt.% DHA, 0.05 wt.% EPA; the LA/ALA ratio is 5.1; (ii) the digestible carbohydrate component comprising 51 gram lactose/100 gram powder; 0.36 g galacto-oligosaccharides with DP 2-6 and 0.4 g fructo-oligosaccharides with DP 7-60; (ii) the protein component comprising cow milk protein. The label of the package of this infant nutrition indicates that the nutrition prevents the development of one or more of the following disorders later-in-life: type 2 diabetes, fasting hyperglycaemia, insulin resistance, visceral adiposity, hyperinsulinemia, hypertension, cardiovascular disease, cerebrovascular disease, artherosclerose, dyslipidaemia, hyperuricaemia, fatty liver, osteoarthritis and/or sleep apnoea.

## Claims

1. Use of a composition comprising a lipid, protein and digestible carbohydrate component wherein the lipid component provides 35 to 55% of the total calories, the protein component provides 5 to 15% of the total calories and the digestible carbohydrate component provides 30 to 60% of the total calories, wherein the lipid component comprises (i) linoleic acid (LA) and alpha-linolenic acid (ALA) in a weight ratio of LA/ALA between 2 and 6; (ii) less than 14.5 wt.% LA based on total fatty acids; (iii) long chain polyunsaturated fatty acids (LC-PUFA); and optionally (iv) 10-50 wt.% medium chain fatty acids (MCFA) for the manufacture of a nutritional composition to be administered to a non-obese human with the age below 36 months and for preventing the development of a disorder when said human has an age above 36 months, wherein said disorder is selected from the group consisting of type 2 diabetes, fasting hyperglycaemia, insulin resistance, visceral adiposity, hyperinsulinemia, hypertension, cardiovascular disease, cerebrovascular disease, arthrosclerosis, dyslipidaemia, hyperuricaemia, fatty liver, osteoarthritis and sleep apnoea.

2. Use according to claim 1, wherein the composition further comprises at least one component selected from the group consisting of fructo-oligosaccharide, galacto-oligosaccharides (including transgalacto-oligosaccharides), casein, lactose, hydrolyzed whey protein and hydrolyzed casein.

3. Use according to claim 1 or 2 wherein the composition comprises docosahexaenoic acid (DHA).

4. Use according to any one of the preceding claims, wherein the composition is a liquid comprising between 0.3 and 0.55 g LA per 100 ml and between 50 and 150 mg ALA per 100 ml.

5. Use according to any one of the preceding claims, wherein at least 90 wt.% of the digestible carbohydrate is lactose.

6. Use according to any one of the preceding claims for preventing the development of a disorder selected from the group consisting of diabetes, visceral adiposity and/or cardiovascular diseases.

7. Use according to any one of the preceding claims, wherein the nutritional composition is administered to a human with the age below 18 months.

8. Use according to any one of the preceding claims, wherein arachidonic acid content does not exceed 0.5 wt.% based on total fatty acids.

9. Use according to any one of the preceding claims, wherein the composition comprises hydrolyzed casein and/or hydrolyzed whey protein.
